(19) Europäisches Patentamt
European Patent Office
Office européen des brevets

(11) Veröffentlichungsnummer: **0 009 683**
**B1**

(12) **EUROPÄISCHE PATENTSCHRIFT**

(45) Veröffentlichungstag der Patentschrift: **10.08.83**

(21) Anmeldenummer: **79103417.6**

(22) Anmeldetag: **12.09.79**

(51) Int. Cl.³: **C 07 C 93/14,** C 07 C 97/10, C 07 D 233/60, A 61 K 31/135, A 61 K 31/415

# (54) Neue basische Phenyläther, diese enthaltende pharmazeutische Zubereitungen und die Herstellung dieser Äther und Zubereitungen.

(30) Priorität: **30.09.78 DE 2842759**

(43) Veröffentlichungstag der Anmeldung: **16.04.80 Patentblatt 80/8**

(45) Bekanntmachung des Hinweises auf die Patenterteilung: **10.08.83 Patentblatt 83/32**

(84) Benannte Vertragsstaaten: **AT BE CH DE FR GB IT NL SE**

(56) Entgegenhaltungen:
**DD-B-9565**
**DE-A-2523565**
**GB-A-787308**
**US-A-2765307**
**US-A-3833603**
**US-A-4006243**

**Die Akte enthält technische Angaben, die nach dem Eingang der Anmeldung eingereicht wurden und die nicht in dieser Patentschrift enthalten sind.**

(73) Patentinhaber: **Merck Patent Gesellschaft mit beschränkter Haftung, Frankfurter Strasse 250, D-6100 Darmstadt (DE)**

(72) Erfinder: **Gante, Joachim, Dr., Stormstrasse 4, D-6100 Darmstadt-Arheilgen (DE)**
Erfinder: **Kurmeier, Hans-Adolf, Dr., Heidelbergerstrasse 37, D-6100 Darmstadt (DE)**
Erfinder: **Orth, Dieter, Dr., Jahnstrasse 83, D-6100 Darmstadt (DE)**
Erfinder: **Prücher, Helmut, Bahnhofstrasse 22, D-6148 Heppenheim (DE)**
Erfinder: **Rudolph, Volkmar, Dr., Im Neutscher Grund 19, D-6104 Seeheim 3 (DE)**
Erfinder: **Wahlig, Helmut, Dr., Roemheldweg 16, D-6100 Darmstadt (DE)**

**EP 0 009 683 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue basische Phenyläther, diese enthaltende pharmazeutische Zubereitungen und die Herstellung dieser Äther und Zubereitungen

Die Erfindung betrifft neue basische Äther der allgemeinen Formel I

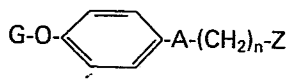 I

worin

- G eine unsubstituierte oder eine ein- oder zweifach durch Halogen substituierte Phenyl- oder Benzylgruppe oder Alkyl mit 1–6 C-Atomen,
- Z 1-Imidazolyl oder 2-Methyl-1-imidazolyl, $-CH=CR^1-$, $-CO-CHR^1-$, $-CHOH-CHR^1-$ oder $-CH_2-CHR^2-$,
- n 1, 2 oder 3,
- $R^1$ H oder Alkyl mit 1–4 C-Atomen und
- $R^2$ Alkyl mit 1–4 C-Atomen

bedeuten, sowie ihre physiologisch unbedenklichen Säureadditionssalze.

Ähnliche Verbindungen sind aus der DT-OS 2510781 bekannt, ferner aus der DE-A-2523565. Weiterhin sind ähnliche Verbindungen in der DD-Z 9565, der GB-A-787308 und der US-A-2765307 beschrieben; diese unterscheiden sich von den Verbindungen der Formel I jedoch dadurch, dass sie an Stelle des Restes Z Dialkylaminogruppen oder heteroaliphatische Gruppen enthalten.

Der Erfindung lag die Aufgabe zugrunde, neue Verbindungen aufzufinden, die zur Herstellung von Arzneimitteln verwendet werden können. Diese Aufgabe wurde durch die Bereitstellung der Verbindungen der Formel I gelöst.

Es wurde gefunden, dass die Verbindungen der Formel I bei guter Verträglichkeit wertvolle pharmakologische Eigenschaften besitzen. Insbesondere treten antimykotische und antibakterielle Wirkungen auf, beispielweise gegen Microsporum audouini, Dermatophyten wie Trichophyton rubrum und Trichophyton mentagrophytes, Histoplasma capsulatum, Aspergillus fumigatus, Hefen wie Candida albicans, Nocardia asteroides, Staphylococcus aureus, Streptococcus pyogenes, Proteus vulgaris, Pseudomonas aeruginosas, Mycobacterium tuberculosis typus humanus, Mycobacterium ranae und/oder Escherichia coli. Die Verbindungen wirken auch gegen systemische Pilzinfektionen; ferner treten Wirkungen gegen Protozonen, insbesondere gegen Trichomonaden, auf.

Diese Wirksamkeiten können z. B. nach der üblichen Agarverdünnungsmethodik in vitro ermittelt werden, aber auch in vivo, beispielsweise an Mäusen, Ratten oder Kaninchen.

Die Verbindungen der Formel I sind beispielsweise gegenüber Candida albicans wirksamer als konstitutionell ähnliche, in der DE-A-2523565 beschriebene Verbindungen, da sie den Teststamm in erheblich kürzeren Zeiten vollständig abtöten.

Weiterhin zeigen sich antiphlogistische Wirkungen, die sich z. B. im Adjuvans-Arthritis-Test nach der Methode von Newbould [Brit. J. Pharmacol. 21. (1963) Seiten 127–136] an Ratten nachweisen lassen. Ferner zeigen sich antiarteriosklerotische, cholesterinspiegelsenkende (nachweisbar im Serum von Ratten nach der Methode von Levine et al. Automation in Analytical Chemistry, Technicon Symposium 1967, Mediad, New York, Seiten 25–28) und triglyceridspiegelsenkende Wirkungen [nachweisbar nach der Methode von Noble and Campbell, Clin. Chem 16 (1970), Seiten 166–170]. Weiterhin können analgetische, antipyretische, enzyminduzierende, fibrinolytische und thrombozyten-aggregationshemmende Wirkungen nach hierfür geläufigen Methoden beobachtet werden.

Die Verbindungen der Formel I und ihre physiologisch unbedenklichen Säureadditionssalze können daher als Arzneimittelwirkstoffe verwendet werden.

In den oben angegebenen Resten bedeutet Alkyl vorzugsweise Methyl oder Äthyl, ferner n-Propyl, Isopropyl, n-Butyl, Isobutyl, sek.-Butyl, tert.-Butyl. Im Rest G kann Alkyl ausserdem beispielsweise bedeuten: 1-, 2- oder 3-Pentyl, 2-Methyl-1-butyl, Isopentyl (3-Methyl-1-butyl), 3-Methyl-2-butyl, tert.-Pentyl, Neopentyl, 1-, 2- oder 3-Hexyl, 2-Methyl-1-, -2- oder -3-pentyl, Isohexyl, (4-Methyl-1-pentyl)- 4-Methyl-2-pentyl, 3-Methyl-1-, -2- oder -3-pentyl, 2-Äthyl-1-butyl, 2,3-Dimethyl-1- oder -2-butyl, 2,2-Dimethyl-1-butyl, 3,3-Dimethyl-1- oder -2-butyl.

Unter Halogen wird vorzugsweise Chlor, aber auch Fluor, Brom oder Jod verstanden.

Im einzelnen bedeutet der Rest $R^1$ vorzugsweise H oder Methyl, der Rest $R^2$ vorzugsweise Methyl.

Der Rest G ist vorzugsweise eine unsubstituierte oder eine 1- oder 2-fach durch Halogen, insbesondere Chlor substituierte Phenyl- oder Benzylgruppe, im einzelnen vorzugsweise Phenyl, p-Chlorphenyl, 2,4-Dichlorphenyl, ferner Benzyl, p-Chlorbenzyl oder 2,4-Dichlorbenzyl, weiterhin z. B. o-, m- oder p-Fluorphenyl, o- oder m-Chlorphenyl, o-, m- oder p-Bromphenyl, o-, m- oder p-Jodphenyl, 2,3-, 2,4-, 2,5-, 2,6-, 3,4- oder 3,5-Difluorphenyl, 2,3-, 2,5-, 2,6-, 3,4- oder 3,5-Dichlorphenyl, Dibromphenyl wie 2,4-Dibromphenyl, Dijodphenyl wie 2,4-Dijodphenyl, Chlor-fluorphenyl wie 2-Fluor-4-chlor-phenyl oder 2-Chlor-4-fluorphenyl, o-, m- oder p-Fluorbenzyl, o-, oder m-Chlorbenzyl, o-, m- oder p-Brombenzyl, o-, m- oder p-Jodbenzyl, 2,3-, 2,4-, 2,5-, 2,6-, 3,4- oder 3,5-Difluorbenzyl, 2,3-, 2,5-, 2,6-, 3,4- oder 3,5-Dichlorbenzyl, 2,3-, 2,4-, 2,5-, 2,6-, 3,4- oder 3,5-Dibrombenzyl, Dijodbenzyl wie 2,4-Dijodbenzyl, Chlor-fluor-benzyl wie 2-Fluor-4-chlor-benzyl oder 2-Chlor-4-fluorbenzyl, Brom-chlorbenzyl wie 2-Brom-4-chlorbenzyl oder 2-Chlor-4-brombenzyl; weiterhin ist G vorzugsweise Methyl, Äthyl, verzweigtes Alkyl wie Isopropyl, Isobutyl (insbesondere), Isopentyl oder Isohexyl.

Der Rest A steht bevorzugt für $-CH=CR^1-$ oder $-CH_2-CHR^2-$, im einzelnen insbesondere für die folgenden Gruppen: $-CH=CH-$, $-CH=C(CH_3)-$, $-CO-CH_2-$, $-CO-CH(CH_3)-$, $-CHOH-CH_2-$, $-CHOH-CH(CH_3)-$ oder $-CH_2CH(CH_3)-$.

Der Parameter n hat vorzugsweise den Wert 1

oder 2, insbesondere 1.

Dementsprechend sind Gegenstand der Erfindung insbesondere diejenigen Verbindungen der Formel I, in denen mindestens einer der Reste G, A und Z bzw. der Parameter n eine der vorstehend angegebenen bevorzugten Bedeutungen hat. Einige bevorzugte Gruppen von Verbindungen können durch die folgenden Teilformeln Ia–Ic ausgedrückt werden, die der Formel I entsprechen und worin die nicht näher bezeichneten Reste die bei der Formel I angegebene Bedeutung haben, worin jedoch

in Ia Z 2-Methyl-1-imidazolyl bedeutet;

in Ib G Phenyl, p-Chlorphenyl oder Isopentyl,
A -CH=CH-, -CH=C($CH_3$)., -CO-$CH_2$-, -CO-CH($CH_3$)-, -CHOH-$CH_2$-, -CHOH-CH($CH_3$)- oder -$CH_2$-CH($CH_3$)- und
n 1 bedeutet;

in Ic G Phenyl oder p-Chlorphenyl,
t A -CH=CH-, -CH=C($CH_3$)- oder -$CH_2$CH($CH_3$),
Z 1-Imidazolyl und
n 1 bedeuten.

Gegenstand der Erfindung ist ferner ein Verfahren zur Herstellung der Verbindungen der Formel I sowie ihrer physiologisch unbedenklichen Säureadditionssalze, dadurch gekennzeichnet, dass man eine Verbindung der allgemeinen Formel II

R–Q II

worin

R die Gruppe G-O-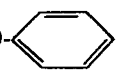 und

Q einen zur Gruppe -A-($CH_2$)$_n$-Z reduzierbaren Rest bedeuten und

G, Z, A und n die oben angegebene Bedeutung haben,

mit einem reduzierenden Mittel behandelt,

oder dass man eine Verbindung, die der allgemeinen Formel I entspricht, worin jedoch die Hydroxygruppe in funktionell abgewandelter Form vorliegt, mit einem solvolysierenden Mittel unter Freisetzung der Hydroxygruppe behandelt,

oder dass man eine Verbindung der allgemeinen Formel III

R-A-($CH_2$)$_n$-X III

worin

X Cl, Br, J, OH oder reaktionsfähig funktionell abgewandeltes OH bedeutet und

R, A und n die oben angegebene Bedeutung haben,

mit einer Verbindung der allgemeinen Formel H-Z umsetzt

und dass man gegebenenfalls eine erhaltene Hydroxyverbiundung der Formel I, in der A -CHOH-CHR$^1$- ist, mit einem Dehydratisierungsmittel und/oder eine erhaltene Verbindung der Formel I, in der A -CH=CR$^1$-, -CO-CHR$^1$ oder -CHOH-CHR$^1$- ist, mit einem reduzierenden Mittel behandelt

und/oder eine erhaltene Base der Formel I durch Behandeln mit einer Säure in eines ihrer physiologisch unbedenklichen Säureadditionssalze umwandelt.

Die Herstellung der Verbindungen der Formel I erfolgt im übrigen nach an sich bekannten Methoden, wie sie in der Literatur (z. B. in den Standardwerken wie Houben-Weyl, Methoden der organischen Chemie, Georg-Thieme-Verlag, Stuttgart) beschrieben sind, und zwar unter den für die genannten Umsetzungen bekannten und geeigneten Reaktionsbedingungen. Dabei kann man auch von an sich bekannten, hier nicht näher erwähnten Varianten Gebrauch machen.

In allen vor- und nachstehenden allgemeinen Formeln haben G, Z, A, n, $R^1$, $R^2$, R, Q und X die bei Formeln I bis III angegebenen Bedeutungen, falls nicht ausdrücklich etwas anderes angegeben ist.

Die Ausgangsstoffe zur Herstellung der Verbindungen der Formel I sind teilweise bekannt. Sie können nach an sich bekannten Verfahren hergestellt werden. Die Ausgangsstoffe können gewünschtenfalls auch in situ gebildet werden, derart, dass man sie aus dem Reaktionsgemisch nicht isoliert, sondern sofort weiter zu den Verbindungen der Formel I umsetzt.

Die Ausgangsstoffe der Formel II sind in der Regel neu; sie können jedoch in Analogie zu bekannten Verfahren hergestellt werden.

Säureamide der Formel R-CHOH-CHR$^1$-CO-Z sind z. B. erhältlich durch Friedel-Crafts-Acylierung oder durch Gattermann- oder Vilsmeier-Reaktion der Äther der Formel R-H zu Aldehyden der Formel R-CHO, Reformatskij-Reaktion mit Estern der Formel Br-CHR$^1$-$COOC_2H_5$ zu Hydroxyestern der Formel R-CHOH-CHR$^1$-$COOC_2H_5$ und Umsetzung mit Basen der Formel HZ. Wasserabspaltung führt zu ungesättigten Amiden der Formel R-CH=CR$^1$-CO-Z, Reduktion (falls $R^1$ = $R^2$) zu gesättigten Amiden der Formel R-$CH_2$CHR$^2$-CO-Z.

Reduktion der Aldehyde der Formel R-CHO liefert die entsprechenden Alkohole der Formel R-$CH_2$OH, die über die entsprechenden Bromide der Formel R-$CH_2$Br leicht in die Nitrile der Formel R-$CH_2$-CN übergeführt werden können. Umsetzung mit Organometallverbindungen der Formel $CH_3$M (worin M Li, MgBr oder MgCl bedeutet) und Hydrolyse liefert Ketone der Formel R-$CH_2$CO-$CH_3$, die durch Bromierung und nachfolgende Umsetzung mit einer Base der Formel H-Z in die Aminoketone der Formel R-$CH_2$-CO-$CH_2$-Z umgewandelt werden können. Diese können durch Reduktion unter Wasserabspaltung in Verbindungen der Formel R-CH=CH-$CH_2$-Z übergeführt werden.

Unter den Ausgangsstoffen der Formel II sind die Amide der Formel R-A-CO-Z bevorzugt.

Die Ausgangsstoffe der Formel II können beispielsweise durch katalytische Hydrierung, mit nascierendem Wasserstoff, mit komplexen Metallhydriden oder mit Hilfe anderer chemischer Reduktionsmittel in die Verbindungen der Formel I umgewandelt werden. Die für die einzelnen Ausgangsstoffe geeignetsten Reduktionsmethoden sind dem Fachmann nach den Angaben der Literatur geläufig. Eine Reduktion der Säureamide erfolgt besonders vorteilhaft mit komplexen Metallhydriden oder mit Diboran.

Für katalytische Hydrierungen eignen sich beispielsweise Edelmetall-, Nickel- oder Kobaltkatalysatoren, ferner auch Mischkatalysatoren wie Kupferchromoxid. Als Edelmetalle kommen in erster Linie Platin und Palladium in Betracht, die auf Trägern (z. B. auf Kohle, Calciumcarbonat oder Strontiumcarbonat), als Oxide (z. B. Platinoxid) oder in feinteiliger Form vorliegen können. Nickel- und Kobaltkatalysatoren werden zweckmässig als Raney-Metalle eingesetzt. Man kann zweckmässig bei Drucken zwischen etwa 1 und 200 at und bei Temperaturen zwischen etwa −80 und +150°, vorzugsweise zwischen 20 und 100° hydrieren. Die Hydrierung erfolgt in Gegenwart eines inerten Lösungsmittels, z. B. eines Alkohols wie Methanol, Äthanol oder Isopropanol, einer Carbonsäure wie Essigsäure, eines Esters wie Äthylacetat, eines Äthers wie Tetrahydrofuran (THF) oder Dioxan. Man kann auch Lösungsmittelgemische verwenden, z. B. auch Wasser enthaltende Gemische. Weiterhin kann es vorteilhaft sein, eine Base wie Natrium- oder Kaliumhydroxid oder Ammoniak bei der Hydrierung zuzufügen, z. B. bei der Hydrierung von Nitrilen.

Ferner können als Reduktionsmittel komplexe Metallhydride wie $LiAlH_4$, $NaBH_4$ oder $NaAl(OCH_2CH_2OCH_3)_2H_2$ sowie Diboran eingesetzt werden, falls erwünscht unter Zusatz von Katalysatoren wie $BF_3$, $AlCl_3$ oder LiBr. Als Lösungsmittel eignen sich hierfür insbesondere Äther wie Diäthyläther, THF, Dioxan, 1,2-Dimethoxyäthan oder Diglyme sowie Kohlenwasserstoffe wie Benzol. Die Reduktion von Ketonen wird bevorzugt mit $NaBH_4$ vorgenommen; dafür sind in erster Linie Alkohole wie Methanol oder Äthanol oder Gemische dieser Alkohole mit THF als Lösungsmittel geeignet. Nach dieser Methode reduziert man vorzugsweise bei Temperaturen zwischen etwa −80 und +150°, insbesondere zwischen etwa 20 und 120°.

Weiterhin ist als Reduktionsmethode die Umsetzung mit nascierendem Wasserstoff geeignet. Dieser kann beispielsweise durch Behandlung von Metallen mit Säuren oder Basen erzeugt werden. So können z. B. die Systeme Zink/Säure, Zink/Alkalilauge, Eisen/Säure oder Zink/Säure verwendet werden. Als Säuren eignen sich z. B. Salzsäure oder Essigsäure. Auch ein Alkalimetall wie Natrium in einem Alkohol wie Äthanol, Isopropanol, n-Butanol, Amylalkohol, Isoamylalkohol oder in Phenol kann als Reduktionsmittel verwendet werden, ferner z. B. eine Aluminium-Nickel-Legierung in alkalisch-wässeriger oder alkalisch-wässerig-alkoholischer Lösung, sowie Natrium- oder Aluminiumamalgam in wässerig-alkoholischer oder wässeriger Lösung. Bei diesen Methoden liegen die Reaktionstemperaturen zwischen etwa 0 und etwa 150°, vorzugsweise zwischen etwa 20 und 120°.

Die Ausgangsverbindungen der Formel II können auch durch kathodische Reduktion in Verbindungen der Formel I umgewandelt werden, zweckmässig in wässerig-alkoholischem oder wässerig-essigsaurem Medium. Weitere geeignete Reduktionsmittel sind beispielsweise Natriumdithionit in wässerig-alkoholischer oder alkalischer Lösung, ferner Eisen(II)-hydroxid, Zinn(II)-chlorid, Schwefelwasserstoff, Hydrogensulfide, Sulfide, Polysulfide, Hydrazin, die sämtliche nach den in der Literatur für derartige Reduktionen angegebenen Bedingungen zur Anwendung kommen.

Die Verbindungen der Formel I sind ferner durch Solvolyse, vorzugsweise Hydrolyse, von Ausgangsstoffen erhältlich, die der Formel I entsprechen, worin jedoch die Hydroxygruppe in funktionell abgewandelter Form vorliegt.

Die Ausgangsstoffe für die Solvolyse sind in der Regel neu, sie können jedoch in Analogie zu an sich bekannten Methoden hergestellt werden.

Bevorzugte Ausgangsstoffe für die Solvolyse, in denen die OH-Gruppe funktionell abgewandelt ist, entsprechen z. B. der allgemeinen Formel IV

$$R\text{-}CHL\text{-}CHR^1\text{-}(CH_2)_n\text{-}Z \qquad IV$$

worin

L eine funktionell abgewandelte OH-Gruppe, insbesondere OM oder OAc und

Ac einen beliebigen Acylrest, dessen Natur nicht kritisch ist, da er bei der Solvolyse abgespalten wird, der jedoch vorzugsweise 1−10 C-Atome besitzt, z. B. Alkanoyl, Aroyl, Alkylsulfonyl oder Arylsulfonyl mit jeweils bis zu 10 C-Atomen, z. B. Acetyl, Benzoyl, Methansulfonyl oder p-Toluolsulfonyl bedeuten und

M die angegebene Bedeutung hat.

Diese Verbindungen sind z. B. die entsprechenden Alkoholate, insbesondere die Magnesium- oder Lithium-Alkoholate, wie sie als Reaktionsprodukte bei Grignard-Reaktionen oder bei Reaktionen mit Organolithiumverbindungen entstehen, und die Ester (z. B. die Carbonsäureester, wobei der Carbonsäurerest vorzugsweise bis zu 7 C-Atomen besitzt, z. B. Acetyl oder Benzoyl, die Alkyl- oder Arylsulfonsäureester, worin der Alkylrest vorzugsweise 1−6, der Arylrest vorzugsweise 6−10 C-Atome enthält), ferner die Äther (z. B. die Alkyläther, worin die Alkylgruppe vorzugsweise bis zu 6 C-Atome enthält, die Aryläther, worin die Arylgruppe vorzugsweise 6-10 C-Atome enthält, und die Aralkyläther, worin die Aralkylgruppe vorzugsweise 7−11 C-Atome besitzt und die Borsäureester, die intermediär bei der oxydativen Hydroborierung entstehen. Weiterhin kann anstelle der Hydroxygruppe ein Chlor-, Brom- oder Jodatom stehen; es liegen dann die entsprechenden Halogenwasserstoffsäureester vor.

Die genannten Magnesium- oder Lithiumalkoholate sind z. B. durch Umsetzung von Organometallverbindungen der Formel R-M mit Aldehyden der Formel $H\text{-}CO\text{-}CHR^1\text{-}(CH_2)_n\text{-}Z$ oder durch Umsetzung von Aldehyden der Formel R-CHO mit Organometallverbindungen der Formel $M\text{-}CHR^1\text{-}(CH_2)_n\text{-}Z$ erhältlich, zweckmässig in einem Äther wie Diäthyläther oder THF als Lösungsmittel. Halogenide der Formeln $R\text{-}CHCl\text{-}CHR^1\text{-}(CH_2)\text{-}_n\text{-}Z$ oder $R\text{-}CHBr\text{-}CHR^1\text{-}(CH_2)_n\text{-}Z$ können z. B. hergestellt werden durch Halogenierung von Säureamiden der Formel $R\text{-}CH_2\text{-}CHR^1(CH_2)_{n\text{-}1}\text{-}CO\text{-}Z$ und anschliessende Reduktion mit $LiAlH_4$. Aus den Halogeniden sind die entsprechenden Ester der Formel $R\text{-}CH(OAc)\text{-}CHR^1\text{-}(CH_2)_n\text{-}Z$ durch Umsetzung mit

Kaliumacylaten, z. B. Kaliumacetat, herstellbar.

Die Solvolyse dieser Verbindungen gelingt zweckmässig durch Einwirkung eines Lösungsmittels wie Wasser (Hydrolyse) oder eines Alkohols mit vorzugsweise 1–4 C-Atomen (Alkoholyse) in Gegenwart eines sauren oder basischen Katalysators, z. B. einer Mineralsäure wie Schwefelsäure oder Salzsäure, eines Metallhydroxids wie Natrium-, Kalium-, Calcium-, Barium-, Blei- oder Silberhydroxid, oder eines Metall- oder Ammoniumsalzes wie Natrium- oder Kaliumcarbonat oder Ammoniumchlorid. Als Alkohole dienen vorzugsweise Methanol, Äthanol oder Isopropanol, man kann auch Gemische von Wasser mit einem dieser Alkohole verwenden. Die Solvolyse erfolgt zweckmässig bei Temperaturen zwischen etwa 0 und etwa 120°.

Im einzelnen werden die genannten Magnesiumalkoholate zweckmässigerweise nicht isoliert, sondern nach ihrer Bildung bei der Grignard-Reaktion in situ mit verdünnten Säuren, z. B. Schwefelsäure oder Salzsäure oder mit wässeriger Ammoniumchloridlösung hydrolysiert. Die genannten Halogenide und Ester werden vorzugsweise in wässeriger oder wässerig-alkoholischer Lösung oder Suspension verseift, wobei, falls erwünscht, ein Lösungsvermittler zugegen sein kann, z. B. ein Alkohol, Glykol oder Glykoläther. Als Verseifungsmittel verwendet man dabei vorzugsweise Alkalien wie NaOH oder KOH.

Die Verbindungen der Formel I sind ferner erhältlich durch Umsetzung einer Verbindung der Formel R-A-$(CH_2)_n$-X (III) mit einer Verbindung der Formel H-Z oder einem reaktionsfähigen Derivat einer solchen Verbindung. Die Ausgangsstoffe der Formel III umschliessen z. B. die Ketone der Formel R-CO-$CHR^1$-$(CH_2)_n$-Cl; sie sind z. B. herstellbar durch Friedel-Crafts-Acylierung der Äther der Formel R-H mit Säurechloriden der Formel Cl-CO-$CHR^1$-$(CH_2)_n$-Cl. Andere Ausgangsstoffe der Formel III können z. B. hergestellt werden durch Reduktion dieser Ketone zu Carbinolen der Formel R-CHOH-$CHR^1$-$(CH_2)$-$_n$-Cl und, falls erwünscht, anschliessende Dehydratisierung und/oder Reduktion. Die Ausgangsstoffe der Formel H-Z sind bekannt.

Die Umsetzung von Verbindungen der Formel III mit Verbindungen der Formel H-Z erfolgt zweckmässig bei Temperaturen zwischen etwa 0 und etwa 250°, vorzugsweise zwischen etwa 50 und 120°, und bei Drucken zwischen etwa 1 und etwa 50 at. Man kann in Gegenwart eines inerten Lösungsmittels arbeiten, z. B. eines Alkohols wie Methanol, Äthanol, Isopropanol, n-Butanol, eines Äthers wie Diäthyläther, Diisopropyläther, THF, Dioxan, eines Kohlenwasserstoffs wie Benzol, Toluol, Xylol, eines Amids wie Dimethylformamid (DMF), eines Sulfoxids wie Dimethylsulfoxid. Falls erwünscht, kann ein Katalysator zugegen sein, z.B. Natriumamid, das auch aus Natrium und flüssigem Ammoniak in situ erzeugt werden kann, ferner Basen wie Natriumcarbonat, Kaliumcarbonat, Natriumbicarbonat oder Kaliumbicarbonat. Es ist auch möglich, einen Überschuss der Verbindung der Formel H-Z als Lösungsmittel zu verwenden, zweckmässig in der Siedehitze. X bedeutet vorzugsweise Cl, Br oder J. Falls X eine reaktionsfähig funktionell abgewandelte OH-Gruppe bedeutet, so steht es vorzugsweise für eine Alkyl- oder Arylsulfonyloxygruppe mit insbesondere bis zu 10 C-Atomen.

Falls erwünscht, kann eine erhaltene Hydroxyverbindung der Formel R-CHOH-$CHR^1$-$(CH_2)$-$_n$-Z zur entsprechenden ungesättigten Verbindung der Formel R-CH=$CR^1$-$(CH_2)_n$-Z dehydratisiert werden, zweckmässig durch Einwirkung eines sauren Katalysators wie Salzsäure, Schwefelsäure oder einer Sulfonsäure wie p-Toluolsulfonsäure in einem inerten Lösungsmittel, z. B. einem Kohlenwasserstoff wie Benzol oder Toluol, bei Temperaturen zwischen etwa 0 und etwa 150°, vorzugsweise zwischen 80 und 110°. Zur Dehydratisierung sekundärer Alkohole verwendet man bevorzugt verdünnte wässrig-äthanolische Salzsäure bei etwa 70–80°, zur Dehydratisierung tertiärer Alkohole dagegen 20%ige wässrige Salzsäure, auch unter Zusatz von Dioxan, bei 90–100°. Bei der Dehydratisierung entstehen in der Regel die (stabileren) trans-Formen (oder E-Formen) der Verbindungen der Formel I (A = -CH=$CR^1$-).

Erhaltene Ketonverbindungen der Formel R-CO-$CHR^1$-$(CH_2)$-$_n$-Z können, falls erwünscht, zu den entsprechenden Hydroxyverbindungen der Formel R-CHOH-$CHR^1$-$(CH_2)$-$_n$-Z reduziert werden, vorzugsweise mit $NaBH_4$ unter den oben angegebenen Bedingungen.

Weiterhin kann man, falls erwünscht, erhaltene ungesättigte Verbindungen der Formel R-CH=$CR^1$-$(CH_2)_n$-Z, Ketoverbindungen der Formel R-CO-$CHR^1$-$(CH_2)_n$-Z oder Hydroxyverbindungen der Formel R-CHOH-$CHR^1$-$(CH_2)_n$-Z, worin jeweils $R^1 = R^2$ ist, zu den gesättigten Verbindungen der Formel R-$CH_2$-$CHR^2$-$(CH_2)_n$-Z reduzieren. Die Reduktion der Hydroxyverbindungen gelingt, z. B. mit Jodwasserstoffsäure, vorzugsweise in Essigsäure bei Temperaturen zwischen 20° und (vorzugsweise) Siedetemperatur. Die Ketoverbindungen und die ungesättigten Verbindungen lassen sich vorzugsweise katalytisch unter den oben angegebenen Bedingungen hydrieren, beispielsweise an einem Edelmetallkatalysator wie Palladium auf Kohle bei Raumtemperatur und Normaldruck.

Eine erhaltene Base der Formel I kann mit einer Säure in üblicher Weise in das zugehörige Säureadditionssalz übergeführt werden. Für diese Umstzung eignen sich Säuren, die physiologisch unbedenkliche Salze liefern. So können anorganische Säuren verwendet werden, z. B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diäthylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- und Äthansulfonsäure, Äthandisulfonsäure, 2-Hydroxyäthansulfonsäure, Benzolsulfonsäure, p-Toluolsulfonsäure, Naphthalin-mono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Die Verbindungen der Formel I können ein oder mehrere Asymmetriezentren enthalten. In diesem Fall liegen sie gewöhnlich in racemischer Form vor. Erhaltene Racemate können nach an sich bekannten Methoden mechanisch oder chemisch in ihre optischen Antipoden getrennt werden. Vorzugsweise werden aus dem racemischen Gemisch durch Umsetzung mit einem optisch-aktiven Trennmittel Diastereomere gebildet. Als Trennmittel eignen sich z. B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch-aktiven Camphersulfonsäuren wie β-Camphersulfonsäure.

Natürlich ist es auch möglich, optisch-aktive Verbindungen der Formel I nach den oben beschriebenen Methoden zu erhalten, indem man Ausgangsstoffe verwendet, die bereits optischaktiv sind.

Gegenstand der Erfindung ist ferner die Verwendung der neuen Verbindungen der Formel I und ihrer physiologisch unbedenklichen Säureadditionssalze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner pharmazeutische Zubereitungen, enthaltend eine Verbindung der Formel I und/oder eines ihrer physiologisch unbedenklichen Säureadditionssalze.

Diese Zubereitungen können als Arzneimittel in der Human- und Veterinärmedizin verwendet werden. Als Trägerstoffe kommen organische oder anorganische Substanzen in Frage, die sich insbesondere für die topikale, aber auch für die enterale (z. B. orale) oder parenterale Applikation eignen und mit den neuen Verbindungen nicht reagieren, beispielsweise Wasser, pflanzliche Öle, Kohlenwasserstoffe wie alkylierte Naphthaline, halogenierte Kohlenwasserstoffe wie $CF_2Cl_2$ (z. B. für Aerosole), Benzylalkohole, Polyäthylenglykole, Glycerintriacetat, Gelatine, Kohlenhydrate wie Lactose oder Stärke, Magnesiumstearat, Talk, Vaseline. Zur oralen Anwendung dienen insbesondere Tabletten, Dragées, Kapseln, Sirupe, Säfte oder Tropfen, zur rektalen Anwendung Suppositorien, zur intravaginalen Ovula, zur parenteralen Anwendung Lösungen, vorzugsweise ölige oder wässrige Lösungen, ferner Suspensionen, Emulsionen oder Implantate, für die topikale Anwendung Lösungen, Lotionen, Emulsionen, Sprays (Aerosole), Salben, Crèmes, Pasten oder Puder. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder mehrere Antibiotika, Vitamine und/oder andere Antimykotika.

Die neuen Verbindungen werden in der Regel in Analogie zu bekannten, im Handel befindlichen Antimykotika (z. B. Clotrimazol oder Miconazol) verabreicht. Bei der bevorzugten topikalen Applikation in Kombination mit dafür geeigneten Trägerstoffen kann eine hohe Aktivität über einen weiten Verdünnungsbereich festgestellt werden. Beispielsweise zeigen sich Konzentrationen des Wirkstoffs zwischen etwa 0,1 und 10 Gew.-%, bezogen auf das Gewicht des verwendeten Präparats, als wirksam für die Bekämpfung von Pilzen oder Bakterien. Bevorzugt sind Konzentrationen von etwa 1 bis 3 Gew.-%.

Sofern die neuen Verbindungen als Antiphlogistika oder Lipidsenker verwendet werden, ist ihre orale Applikation bevorzugt. Sie werden dann in der Regel in Analogie zu bekannten Antiphlogistika (z. B. Indometacin) oder Lipidsenkern (z. B. Clofibrat) verabreicht, vorzugsweise in Dosierungen zwischen etwa 5 und 500 mg, insbesondere zwischen 20 und 200 mg pro Dosierungseinheit. Die tägliche Dosierung liegt vorzugsweise zwischen etwa 0,2 und 20 mg/kg Körpergewicht. Die spezielle Dosis für jeden Patienten hängt jedoch von den verschiedensten Faktoren ab, z. B. von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidunggeschwindigkeit, Arzneistoffkombination und Schwere der jeweiligen Erkrankung. So können in Einzelfällen auch höhere oder niedrigere Konzentrationen bzw. Dosierungen als die angegebenen angewendet werden.

Jede der in den folgenden Beispielen genannten Verbindungen der Formel I ist zur Herstellung von pharmazeutischen Zubereitungen besonders geeignet.

In den nachfolgenden Beispielen bedeutet «übliche Aufarbeitung»: Man gibt, falls erforderlich, Wasser oder verdünnte Natronlauge hinzu, extrahiert mit einem organischen Lösungsmittel, das mit Wasser nicht mischbar ist (z. B. Benzol, Chloroform oder Dichlormethan), trennt ab, trocknet die organische Phase über Natriumsulfat, filtriert, dampft ein und reinigt durch Chromatographie und/oder Kristallisation. Das Produkt kann auch durch Kristallisation eines seiner Säureadditionssalze gereinigt werden.

Beispiel 1

Man tropft 100 ml einer 1-molaren Lösung von 2-(1-Imidazolyl)-äthyllithium in Äther unter $N_2$ und Rühren zu einer siedenden Lösung von 21,7 g 4-p-Chlorphenoxy-benzaldehyd in 350 ml Äther, rührt noch eine h bei 25° und 2,5 h bei 35° und zersetzt das gebildete Lithium-1-(4-p-chlorphenoxyphenyl)-3-(1-imidazolyl)-propan-1-olat durch Zugabe von 200 ml gesättigter $NH_4Cl$-Lösung unter Eiskühlung. Nach üblicher Aufarbeitung erhält man 1-(4-p-Chlorphenoxy-phenyl)-3-(1-imidazolyl)-propan-1-ol, F. 102–103°.

Beispiel 2

Man versetzt eine Grignard-Lösung aus 24,9 g p-Bromdiphenyläther und 2,43 g Magnesium in 1000 ml Äther tropfenweise mit 12,4 g 3-(1-Imidazolyl)-propanal (erhältlich durch Reaktion von 3-Chlorpropanal-diäthylacetal mit Imidazol und nachfolgende Hydrolyse) in 400 ml Äther unter Rühren bei 20°, rührt noch 2 h, zersetzt das erhaltene Alkoholat mit verdünnter Schwefelsäure, arbeitet wie üblich auf und erhält 1-p-Phenoxyphenyl-3-(1-imidazolyl)-propan-1-ol.

Beispiel 3

Ein Gemisch aus 2,75 g 1-p-Phenoxyphenyl-2-methyl-3-chlorpropan-1-on (erhältlich durch Friedel-Crafts-Reaktion von Diphenyläther mit 2-Methyl-3-chlorpropionyl-chlorid) und 1,36 g Imidazol wird 3 h auf 140° erhitzt. Nach dem Abkühlen und üblicher Aufarbeitung erhält man 1-p-Phenoxyphenyl-2-methyl-3-(1-imidazolyl)-propan-1-on, Hydrochlorid, F. 149–151Gew.-%.

Beispiele 4 bis 41

Analog Beispiel 3 erhält man aus den entsprechenden Chlor- oder Bromketonen mit Imidazol oder 2-Methylimidazol:

4. 1-p-Phenoxyphenyl-3-(1-imidazolyl)-propan-1-on.
5. 1-(4-p-Chlorphenoxy-phenyl)-3-(1-imidazolyl)-propan-1-on, Hydrochlorid, F. 157–159°.
6. 1-[4-(2,4-Dichlorphenoxy)-phenyl]-3-(1-imidazolyl)-propan-1-on.
7. 1-p-Isopentyloxyphenyl-3-(1-imidazolyl)-propan-1-on.
8. 1-(4-p-Fluorphenoxy-phenyl)-2-methyl-3-(1-imidazolyl)-propan-1-on.
9. 1-(4-o-Chlorphenoxy-phenyl)-2-methyl-3-(1-imidazolyl)-propan-1-on.
10. 1-(4-m-Chlorphenoxy-phenyl)-2-methyl-3-(1-imidazolyl)-propan-1-on.
11. 1-(4-p-Chlorphenoxy-phenyl)-2-methyl-3-(1-imidazolyl)-propan-1-on.
12. 1-(4-p-Bromphenoxy-phenyl)-2-methyl-3-(1-imidazolyl)-propan-1-on.
13. 1-(4-p-Jodphenoxy-phenyl)-2-methyl-3-(1-imidazolyl)-propan-1-on.
14. 1-[4-(2,4-Dichlorphenoxy)-phenyl]-2-methyl-3-(1-imidazolyl)-propan-1-on.
15. 1-p-Benzyloxyphenyl-2-methyl-3-(1-imidazolyl)-propan-1-on.
16. 1-(4-p-Chlorbenzyloxy-phenyl)-2-methyl-3-(1-imidazolyl)-propan-1-on.
17. 1-[4-(2,4-Dichlorbenzyloxy)-phenyl]-2-methyl-3-(1-imidazolyl)-propan-1-on.
18. 1-p-Methoxyphenyl-2-methyl-3-(1-imidazolyl)-propan-1-on.
19. 1-p-Isopentyloxyphenyl-2-methyl-3-(1-imidazolyl)-propan-1-on.
20. 1-p-Isohexyloxyphenyl-2-methyl-3-(1-imidazolyl)-propan-1-on.
21. 1-p-Phenoxyphenyl-2-n-butyl-3-(1-imidazolyl)-propan-1-on.
22. 1-p-Phenoxyphenyl-4-(1-imidazolyl)-butan-1-on.
23. 1-(4-p-Chlorphenoxy-phenyl)-4-(1-imidazolyl)-butan-1-on.
24. 1-p-Phenoxyphenyl-2-methyl-4-(1-imidazolyl)-butan-1-on.
25. 1-(4-p-Chlorphenoxy-phenyl)-2-methyl-4-(1-imidazolyl)-butan-1-on.
26. 1-p-Phenoxyphenyl-5-(1-imidazolyl)-pentan-1-on.
27. 1-(4-p-Chlorphenoxy-phenyl)-5-(1-imidazolyl)-pentan-1-on.
28. 1-p-Phenoxyphenyl-3-(2-methyl-1-imidazolyl)-propan-1-on.
29. 1-(4-p-Chlorphenoxy-phenyl)-3-(2-methyl-1-imidazolyl)-propan-1-on.
30. 1-[4-(2,4-Dichlorphenoxy)-phenyl]-3-(2-methyl-1-imidazolyl)-propan-1-on.
31. 1-p-Isopentyloxyphenyl-3-(2-methyl-1-imidazolyl)-propan-1-on.
32. 1-p-Phenoxyphenyl-2-methyl-3-(2-methyl-1-imidazolyl)-propan-1-on.
33. 1-(4-p-Chlorphenoxy-phenyl)-2-methyl-3-(2-methyl-1-imidazolyl)-propan-1-on.
34. 1-[4-(2,4-Dichlorphenoxy)-phenyl]-2-methyl-3-(2-methyl-1-imidazolyl)-propan-1-on.
35. 1-p-Benzyloxyphenyl-2-methyl-3-(2-methyl-1-imidazolyl)-propan-1-on.
36. 1-(4-p-Chlorbenzyloxy-phenyl)-2-methyl-3-(2-methyl-1-imidazolyl)-propan-1-on.
37. 1-[4-(2,4-Dichlorbenzyloxy)-phenyl]-3-(2-methyl-1-imidazolyl)-propan-1-on.
38. 1-p-Methoxyphenyl-3-(2-methyl-1-imidazolyl)-propan-1-on.
39. 1-p-Isopentyloxyphenyl-3-(2-methyl-1-imidazolyl)-propan-1-on.
40. 1-p-Phenoxyphenyl-4-(2-methyl-1-imidazolyl)-butan-1-on.
41. 1-(4-p-Chlorphenoxyphenyl)-4-(2-methyl-1-imidazolyl)-butan-1-on.

Beispiel 42

Ein Gemisch aus 3,06 g 1-p-Phenoxyphenyl-2-methyl-3-(1-imidazolyl)-propan-1-on, 1,5 g KOH, 2,5 ml 85%igem Hydrazin und 25 ml Diäthylenglykol wird 1 h auf 100° erwärmt. Man steigert die Temperatur langsam bis zur Zersetzung des Hydrazons, kocht noch 4 h, kühlt ab, arbeitet wie üblich auf und erhält 1-p-Phenoxyphenyl-2-methyl-3-(1-imidazolyl)-propan, Hydrochlorid, F. 146–148°.

Beispiel 43

Man gibt portionsweise 4,15 g $NaBH_4$ zu einer Lösung von 30,6 g 1-p-Phenoxyphenyl-2-methyl-3-(1-imidazolyl)-propan-1-on in 160 ml Methanol und 160 ml THF unter Rühren, rührt noch 1 h bei 20°, verdünnt mit Eiswasser, arbeitet wie üblich auf und erhält 1-p-Phenoxyphenyl-2-methyl-3-(1-imidazolyl)-propan-1-ol.

Beispiele 44 bis 81

Analog Beispiel 43 erhält man aus den entsprechenden Ketonen mit $NaBH_4$:

44. 1-p-Phenoxyphenyl-3-(1-imidazolyl)-propan-1-ol.
45. 1-(4-p-Chlorphenoxy-phenyl)-3-(1-imidazolyl)-propan-1-ol, F. 102–103°.
46. 1-[4-(2,4-Dichlorphenoxy)-phenyl]-3-(1-imidazolyl)-propan-1-ol.
47. 1-p-Isopentyloxyphenyl-3-(1-imidazolyl)-propan-1-ol.
48. 1-(4-p-Fluorphenoxy-phenyl)-2-methyl-3-(1-imidazolyl)-propan-1-ol.
49. 1-(4-o-Chlorphenoxy-phenyl)-2-methyl-3-(1-imidazolyl)-propan-1-ol.
50. 1-(4-m-Chlorphenoxy-phenyl)-2-methyl-3-(1-imidazolyl)-propan-1-ol.
51. 1-(4-p-Chlorphenoxy-phenyl)-2-methyl-3-(1-imidazolyl)-propan-1-ol, F. 159–162°.
52. 1-(4-p-Bromphenoxy-phenyl)-2-methyl-3-(1-imidazolyl)-propan-1-ol.
53. 1-(4-p-Jodphenoxy-phenyl)-2-methyl-3-(1-imidazolyl)-propan-1-ol.
54. 1-[4-(2,4-Dichlorphenoxy)-phenyl]-2-methyl-3-(1-imidazolyl)-propan-1-ol.
55. 1-p-Benzyloxyphenyl-2-methyl-3-(1-imidazolyl)-propan-1-ol.
56. 1-(4-p-Chlorbenzyloxy-phenyl)-2-methyl-3-(1-imidazolyl)-propan-1-ol.
57. 1-[4-(2,4-Dichlorbenzyloxy)-phenyl]-2-methyl-3-(1-imidazolyl)-propan-1-ol.
58. 1-p-Methoxyphenyl-2-methyl-3-(1-imidazolyl)-propan-1-ol.
59. 1-p-Isopentyloxyphenyl-2-methyl-3-(1-imidazolyl)-propan-1-ol.
60. 1-p-Isohexyloxyphenyl-2-methyl-3-(1-imidazolyl)-propan-1-ol.
61. 1-p-Phenoxyphenyl-2-n-butyl-3-(1-imidazolyl)-propan-1-ol.
62. 1-p-Phenoxyphenyl-4-(1-imidazolyl)-butan-1-ol.
63. 1-(4-p-Chlorphenoxy-phenyl)-4-(1-imidazolyl)-butan-1-ol.
64. 1-p-Phenoxyphenyl-2-methyl-4-(1-imidazolyl)-butan-1-ol.
65. 1-(4-p-Chlorphenoxy-phenyl)-2-methyl-4-(1-imidazolyl)-butan-1-ol.
66. 1-p-Phenoxyphenyl-5-(1-imidazolyl)-pentan-1-ol.
67. 1-(4-p-Chlorphenoxy-phenyl-5-(1-imidazolyl)-pentan-1-ol.
68. 1-p-Phenoxyphenyl-3-(2-methyl-1-imidazolyl)-propan-1-ol.
69. 1-(4-p-Chlorphenoxy-phenyl)-3-(2-methyl-1-imidazolyl)-propan-1-ol.
70. 1-[4-(2,4-Dichlorphenoxy)-phenyl]-3-(2-methyl-1-imidazolyl)-propan-1-ol.
71. 1-p-Isopentyloxyphenyl-3-(2-methyl-1-imidazolyl)-propan-1-ol.
72. 1-p-Phenoxyphenyl-2-methyl-3-(2-methyl-1-imidazolyl)-propan-1-ol.
73. 1-(4-p-Chlorphenoxy-phenyl)-2-methyl-3-(2-methyl-1-imidazolyl)-propan-1-ol, Fumarat, F. 175–177°.
74. 1-[4-(2,4-Dichlorphenoxy)-phenyl]-2-methyl-3-(2-methyl-1-imidazolyl)-propan-1-ol.
75. 1-p-Benzyloxyphenyl-2-methyl-3-(2-methyl-1-imidazolyl)-propan-1-ol.
76. 1-(4-p-Chlorbenzyloxy-phenyl)-2-methyl-3-(2-methyl-1-imidazolyl)-propan-1-ol.
77. 1-[4-(2,4-Dichlorbenzyloxy)-phenyl]-2-methyl-3-(2-methyl-1-imidazolyl)-propan-1-ol.
78. 1-p-Methoxyphenyl-2-methyl-3-(2-methyl-1-imidazolyl)-propan-1-ol.
79. 1-p-Isopentyloxyphenyl-2-methyl-3-(2-methyl-1-imidazolyl)-propan-1-ol.
80. 1-p-Phenoxyphenyl-4-(2-methyl-1-imidazolyl)-butan-1-ol.
81. 1-(4-p-Chlorphenoxy-phenyl)-4-(2-methyl-1-imidazolyl)-butan-1-ol.

Beispiel 82

Man kocht 29,4 g 1-p-Phenoxyphenyl-3-(1-imidazolyl)-propan-1-ol mit 10 g p-Toluolsulfonsäure in 500 ml Toluol 2 h mit Wasserabscheider, kühlt ab, arbeitet mit Natronlauge auf und erhält 1-p-Phenoxyphenyl-3-(1-imidazolyl)-propen, Hydrochlorid, F. 148–151°.

Beispiel 83

Ein Gemisch aus 3,43 g 1-(4-p-Chlorphenoxy-phenyl)-2-methyl-3-(1-imidazolyl)-propan-1-ol, 0,1 g Benzolsulfonsäure und 80 ml Benzol wird 24 h mit Wasserabscheider gekocht. Nach üblicher Aufarbeitung erhält man 1-(4-p-Chlorphenoxy-phenyl)-2-methyl-3-(1-imidazolyl)-propen, Hydrochlorid, F. 131–133°. Methansulfonat, F. 153–155°.

Beispiel 84

Man löst 1-(4-p-Chlorphenoxy-phenyl)-3-(1-imidazolyl)-propan-1-ol in 240 ml Äthanol, versetzt mit 30 ml 37%iger wässriger Salzsäure, kocht das Gemisch 1 h und dampft ein. Nach üblicher Aufarbeitung erhält man 1-(4-p-Chlorphenoxy-phenyl)-3-(1-imidazolyl)-propen, Hydrochlorid, F. 147–149°.

Beispiel 85

Man erhitzt 30,8 g 1-p-Phenoxyphenyl-2-methyl-3-(1-imidazolyl)-propan-1-ol mit 325 ml 20%iger wässeriger Salzsäure 45 min auf 100°, dampft ein, arbeitet wie üblich auf und erhält 1-p-Phenoxyphenyl-2-methyl-3-(1-imidazolyl)-propen, Hydrochlorid, F. 160–162°.

Beispiele 86 bis 120

Aus den entsprechenden Alkoholen erhält man analog Beispiele 82, 83, 84 oder 85 die folgenden

Alkene (wobei man zur besseren Lösung auch noch etwas Dioxan zugeben kann):

86. 1-[4-(2,4-Dichlorphenoxy)-phenyl]-3-(1-imidazolyl)-propen.
87. 1-p-Isopentyloxyphenyl-3-(1-imidazolyl)-propen.
88. 1-(4-p-Fluorphenoxy-phenyl)-2-methyl-3-(1-imidazolyl)-propen.
89. 1-(4-o-Chlorphenoxy-phenyl)-2-methyl-3-(1-imidazolyl)-propen.
90. 1-(4-m-Chlorphenoxy-phenyl)-2-methyl-3-(1-imidazolyl)-propen.
91. 1-(4-p-Bromphenoxy-phenyl)-2-methyl-3-(1-imidazolyl)-propen.
92. 1-(4-p-Jodphenoxy-phenyl)-2-methyl-3-(1-imidazolyl)-propen.
93. 1-[4-(2,4-Dichlorphenoxy)-phenyl]-2-methyl-3-(1-imidazolyl)-propen.
94. 1-p-Benzyloxyphenyl-2-methyl-3-(1-imidazolyl)-propen.
95. 1-(4-p-Chlorbenzyloxy)-phenyl-2-methyl-3-(1-imidazolyl)-propen.
96. 1-[4-(2,4-Dichlorbenzyloxy)-phenyl]-2-methyl-3-(1-imidazolyl)-propen.
97. 1-p-Methoxyphenyl-2-methyl-3-(1-imidazolyl)-propen.
98. 1-p-Isopentyloxyphenyl-2-methyl-3-(1-imidazolyl)-propen.
99. 1-p-Isohexyloxyphenyl-2-methyl-3-(1-imidazolyl)-propen.
100. 1-p-Phenoxyphenyl-2-n-butyl-3-(1-imidazolyl)-propen.
101. 1-p-Phenoxyphenyl-4-(1-imidazolyl)-buten.
102. 1-(4-p-Chlorphenoxy-phenyl)-4-(1-imidazolyl)-propen.
103. 1-p-Phenoxyphenyl-2-methyl-4-(1-imidazolyl)-buten.
104. 1-(4-p-Chlorphenoxy-phenyl)-2-methyl-4-(1-imidazolyl)-buten.
105. 1-p-Phenoxyphenyl-5-(1-imidazolyl)-penten.
106. 1-(4-p-Chlorphenoxy-phenyl)-5-(1-imidazolyl)-penten.
107. 1-p-Phenoxyphenyl-3-(2-methyl-1-imidazolyl)-propen.
108. 1-(4-p-Chlorphenoxy-phenyl-3-(2-methyl-1-imidazolyl)-propen.
109. 1[4-(2,4-Dichlorphenoxy)-phenyl]-3-(2-methyl-1-imidazolyl)-propen.
110. 1-p-Isopentyloxyphenyl-3-(2-methyl-1-imidazolyl)-propen.
111. 1-p-Phenoxyphenyl-2-methyl-3-(2-methyl-1-imidazolyl)-propen, Hydrochlorid, F. 179–181°.
112. 1-(4-p-Chlorphenoxy-phenyl)-2-methyl-3-(2-methyl-1-imidazolyl)-propen, Hydrochlorid, F. 175–178°.
113. 1-[4-(2,4-Dichlorphenoxy)-phenyl]-2-methyl-3-(2-methyl-1-imidazolyl)-propen.
114. 1-p-Benzyloxyphenyl-2-methyl-3-(2-methyl-1-imidazolyl)-propen.
115. 1-(4-p-Chlorbenzyloxy-phenyl)-2-methyl-3-(2-methyl-1-imidazolyl)-propen.
116. 1-[4-(2,4-Dichlorbenzyloxy)-phenyl]-2-methyl-3-(2-methyl-1-imidazolyl)-propen.
117. 1-p-Methoxyphenyl-2-methyl-3-(2-methyl-1-imidazolyl)-propen.
118. 1-p-Isopentyloxyphenyl-2-methyl-3-(2-methyl-1-imidazolyl)-propen.
119. 1-p-Phenoxyphenyl-4-(2-methyl-1-imidazolyl)-buten.
120. 1-(4-p-Chlorphenoxy-phenyl)-2-(2-methyl-1-imidazolyl)-buten.

Beispiel 121

Ein Gemisch aus 3,43 g 1-(4-p-Chlorphenoxy-phenyl)-2-methyl-3-(1-imidazolyl)-propan-1-ol, 10 ml 67%iger wässeriger Jodwasserstoffsäure und 18 ml Essigsäure wird 1,5 h auf 150° erhitzt. Nach Abkühlen und üblicher Aufarbeitung erhält man 1-(4-p-Chlorphenoxy-phenyl)-2-methyl-3-(1-imidazolyl)-propan, F. 113–116°. Methansulfonat, F. 123–125°.

Beispiele 122 bis 145

Analog Beispiel 121 erhält man durch Reduktion der entsprechenden Hydroxyamine:

122. 1-p-Phenoxyphenyl-2-methyl-3-(1-imidazolyl)-propan, Hydrochlorid, F. 146–148°.
123. 1-(4-p-Fluorphenoxy-phenyl)-2-methyl-3-(1-imidazolyl)-propan.
124. 1-(4-o-Chlorphenoxy-phenyl)-2-methyl-3-(1-imidazolyl)-propan.
125. 1-(4-m-Chlorphenoxy-phenyl)-2-methyl-3-(1-imidazolyl)-propan.
126. 1-(4-p-Bromphenoxy-phenyl)-2-methyl-3-(1-imidazolyl)-propan.
127. 1-(4-p-Jodphenoxy-phenyl)-2-methyl-3-(1-imidazolyl)-propan.
128. 1-[4-(2,4-Dichlorphenoxy)-phenyl]-2-methyl-3-(1-imidazolyl)-propan.
129. 1-p-Benzyloxyphenyl-2-methyl-3-(1-imidazolyl)-propan.
130. 1-(4-p-Chlorbenzyloxy-phenyl)-2-methyl-3-(1-imidazolyl)-propan.
131. 1-[4-(2,4-Dichlorbenzyloxy)-phenyl]-2-methyl-3-(1-imidazolyl)-propan.
132. 1-p-Methoxyphenyl-2-methyl-3-(1-imidazolyl)-propan.
133. 1-p-Isopentyloxyphenyl-2-methyl-3-(1-imidazolyl)-propan, Öl.
134. 1-p-Isohexyloxyphenyl-2-methyl-3-(1-imidazolyl)-propan.
135. 1-p-Phenoxyphenyl-2-n-butyl-3-(1-imidazolyl)-propan.
136. 1-p-Phenoxyphenyl-2-methyl-4-(1-imidazolyl)-butan.
137. 1-(4-p-Chlorphenoxy-phenyl)-2-methyl-4-(1-imidazolyl)-butan.
138. 1-p-Phenoxyphenyl-2-methyl-3-(2-methyl-1-imidazolyl)-propan, Hydrochlorid, F. 155–157°.
139. 1-(4-p-Chlorphenoxy-phenyl)-2-methyl-3-(2-methyl-1-imidazolyl)-propan, Hydrochlorid, F. 145–148°.
140. 1-[4-(2,4-Dichlorphenoxy)-phenyl]-2-methyl-3-(2-methyl-1-imidazolyl)-propan.
141. 1-p-Benzyloxyphenyl-2-methyl-3-(2-methyl-1-imidazolyl)-propan.
142. 1-(4-p-Chlorbenzyloxy-phenyl)-2-methyl-3-(2-methyl-1-imidazolyl)-propan.
143. 1-[4-(2,4-Dichlorbenzyloxy)-phenyl]-2-methyl-3-(2-methyl-1-imidazolyl)-propan.

144. 1-p-Methoxyphenyl-2-methyl-3-(2-methyl-1-imidazolyl)-propan.
145. 1-p-Isopentyloxyphenyl-2-methyl-3-(2-methyl-1-imidazolyl)-propan.

Beispiel 146

Eine Lösung von 29 g 1-p-Phenoxyphenyl-2-methyl-3-(1-imidazolyl)-propen in 500 ml Methanol wird an 10 g 5%igem Pd-C bei 20° und Normaldruck bis zum Ende der Wasserstoffaufnahme hydriert. Man filtriert, dampft ein und erhält 1-p-Phenoxyphenyl-2-methyl-3-(1-imidazolyl)-propan, Hydrochlorid, F. 146–148°.

Beispiele 147 bis 150

Analog Beispiel 3 erhält man aus 1-(4-o-Chlorphenoxy-phenyl)-3-chlor-propan-1-on bzw. aus 1-(4-o-Chlorphenoxy-phenyl)-2-methyl-3-chlor-propan-1-on mit Imidazol oder 2-Methylimidazol:
147. 1-(4-o-Chlorphenoxy-phenyl)-3-(1-imidazolyl)-propan-1-on.
148. 1-(4-o-Chlorphenoxy-phenyl)-2-methyl-3-(1-imidazolyl)-propan-1-on.
149. 1-(4-o-Chlorphenoxy-phenyl)-3-(2-methyl-1-imidazolyl)-propan-1-on.
150. 1-(4-o-Chlorphenoxy-phenyl)-2-methyl-3-(2-methyl-1-imidazolyl)-propan-1-on.

Beispiele 151 bis 154

Analog Beispiel 43 erhält man aus den entsprechenden Ketonen mit $NaBH_4$:
151. 1-(4-o-Chlorphenoxy-phenyl)-3-(1-imidazolyl)-propan-1-ol.
152. 1-(4-o-Chlorphenoxy-phenyl)-2-methyl-3-(1-imidazolyl)-propan-1-ol.
153. 1-(4-o-Chlorphenoxy-phenyl)-3-(2-methyl-1-imidazolyl)-propan-1-ol.
154. 1-(4-o-Chlorphenoxy-phenyl)-2-methyl-3-(2-methyl-1-imidazolyl)-propan-1-ol.

Beispiele 155 bis 158

Analog Beispiel 82 erhält man durch Dehydratisierung der entsprechenden Alkohole:
155. 1-(4-o-Chlorphenoxy-phenyl)-3-(1-imidazolyl)-propen.
156. 1-(4-o-Chlorphenoxy-phenyl)-2-methyl-3-(1-imidazolyl)-propen.
157. 1-(4-o-Chlorphenoxy-phenyl)-3-(2-methyl-1-imidazolyl)-propen.
158. 1-(4-o-Chlorphenoxy-phenyl)-2-methyl-3-(2-methyl-1-imidazolyl)-propen.

Beispiele 159 und 160

Analog Beispiel 121 erhält man durch Reduktion der entsprechenden Alkohole:
159. 1-(4-o-Chlorphenoxy-phenyl)-2-methyl-3-(1-imidazolyl)-propan.
160. 1-(4-o-Chlorphenoxy-phenyl)-2-methyl-3-(2-methyl-1-imidazolyl)-propan.

Die nachstehenden Beispiele betreffen pharmazeutische Zubereitungen, die Äther der Formel I oder ihre Säureadditionssalze enthalten:

Beispiel A: Tabletten

Ein Gemisch von 1 kg 1-(4-p-Chlorphenoxy-phenyl-3-(1-imidazolyl)-propen-hydrochlorid, 4 kg Lactose, 1,2 kg Kartoffelstärke, 0,2 kg Talk und 0,1 kg Magnesiumstearat wird in üblicher Weise zu Tabletten gepresst, derart, dass jede Tablette 50 mg Wirkstoff enthält.

Beispiel B: Dragées

Analog Beispiel A werden Tabletten gepresst, die anschliessend in üblicher Weise mit einem Überzug aus Saccharose, Kartoffelstärke, Talk, Tragant und Farbstoff überzogen werden.

Beispiel C: Kapseln

10 kg 1-p-Phenoxyphenyl-2-methyl-3-(1-imidazolyl)-propen-hydrochlorid werden in üblicher Weise in Hartgelatinekapseln gefüllt, so dass jede Kapsel 50 mg Wirkstoff enthält.

Beispiel D: Ampullen

Eine Lösung von 1 kg 1-p-Phenoxyphenyl-2-methyl-3-(1-imidazolyl)-propan-hydrochlorid in 30 l zweifach destilliertem Wasser wird steril filtriert, in Ampullen abgefüllt, unter sterilen Bedingungen lyophilisiert und steril verschlossen. Jede Ampulle enthält 20 mg Wirkstoff.

Beispiel E: Salbe

Man löst 2 kg 1-(4-p-Chlorphenoxy-phenyl)-2-methyl-3-(1-imidazolyl)-propan-hydrochlorid in einem warmen verflüssigten Gemisch von 40 kg Polyäthylenglykol 400 und 58 kg Polyäthylenglykol 1500. Die Lösung wird während des Kühlens gerührt und als Salbe zur Behandlung von Pilz- und Bakterieninfektionen verwendet.

Beispiel F: Crème

Man erwärmt in üblicher Weise ein Gemisch aus 20 kg 1-(4-p-Chlorphenoxy-phenyl)-2-methyl-3-(1-imidazolyl)-propen-hydrochlorid, 200 kg Polyäthylenglykol 1000-monocetyläther, 50 kg Polyäthylenglykol 1500 -mono-cetyläther, 150 kg Vaseline, 50 kg Paraffinöl und 2 kg Sorbinsäure, lässt abkühlen und rührt 528 kg Wasser ein.

Beispiel G: Crème

Man erwärmt ein Gemisch aus 1-(4-p-Chlorphenoxy-phenyl)-2-methyl-3-(1-imidazolyl)-propen-hydrochlorid, 5 kg 1,2-Propandiol, 5 kg Glycerinstearat, 5 kg Walrat, 10 kg Isopropylmyristat und 4 kg Polysorbat 60, lässt abkühlen und rührt 69 kg Wasser ein.

Beispiel H: Lösung

2 kg 1-(4-p-Chlorphenoxy-phenyl-(2-methyl-3-(1-imidazolyl)-propan-hydrochlorid werden in 98 kg 1,2-Propandiol gelöst. Die Lösung wird zur Behandlung von Pilz- und Bakterieninfektionen verwendet.

Beispiel I: Spray

Der Spray besteht aus einer Lösung von 1 (Gewichtsteil) 1-(4-p-Chlorphenoxy-phenyl)-2-methyl-3-(1-imidazolyl)-propen-hydrochlorid, 10 Isopropylmyristat, 15 Paraffinöl, 30 Äthanol und 44 Isopropanol.

Analog sind Tabletten, Dragées, Kapseln, Ampullen, Salben, Crèmes, Lösungen und Sprays erhältlich, die einen oder mehrere der übrigen Wirkstoffe der Formel I und/oder ihrer physiologisch unbedenklichen Salze enthalten.

**Patentansprüche für die Vertragsstaaten: BE, CH, DE, FR, GB, IT, NL, SE**

1. Basische Äther der allgemeinen Formel I

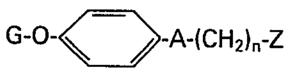 I

worin
G eine unsubstituierte oder eine ein- oder zweifach durch Halogen substituierte Phenyl- oder Benzylgruppe oder Alkyl mit 1–6 C-Atomen,
Z 1-Imidazolyl oder 2-Methyl-1-imidazolyl,
A $-CH=CR^1-$, $-CO-CHR^1-$, $-CHOH-CHR^1-$ oder $-CH_2-CHR^2-$,
n 1, 2 oder 3,
$R^1$ H oder Alkyl mit 1–4 C-Atomen und
$R^2$ Alkyl mit 1–4 C-Atomen
bedeuten sowie ihre physiologisch unbedenklichen Säureadditionssalze.

2. 1-(4-p-Chlorphenoxy-phenyl)-3-(1-imidazolyl)-propen, eine Verbindung nach Anspruch 1.

3. 1-p-Phenoxyphenyl-2-methyl-3-(1-imidazolyl)-propen, eine Verbindung nach Anspruch 1.

4. 1-p-Phenoxyphenyl-2-methyl-3-(1-imidazolyl)-propan, eine Verbindung nach Anspruch 1.

5. 1-(4-p-Chlorphenoxy-phenyl)-2-methyl-3-(1-imidazolyl)-propan, eine Verbindung nach Anspruch 1.

6. 1-(4-p-Chlorphenoxy-phenyl)-2-methyl-3-(1-imidazolyl)-propen, eine Verbindung nach Anspruch 1.

7. Verfahren zur Herstellung von basischen Äthern der allgemeinen Formel I

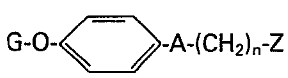 I

worin
G eine unsubstituierte oder eine ein- oder zweifach durch Halogen substituierte Phenyl- oder Benzylgruppe oder Alkyl mit 1–6 C-Atomen,
Z 1-Imidazolyl oder 2-Methyl-1-imidazolyl,
A $-CH=CR^1.$, $-CO-CHR^1-$, $-CHOH-CHR^1-$ oder $-CH_2-CHR^2-$,
n 1, 2 oder 3,
$R^1$ H oder Alkyl mit 1–4 C-Atomen und
$R^2$ Alkyl mit 1–4 C-Atomen
bedeuten sowie ihrer physiologisch unbedenklichen Säureadditionssalze, dadurch gekennzeichnet, dass man eine Verbindung der allgemeinen Formel II

R–Q II

worin
R die Gruppe 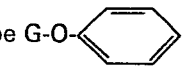 und
Q einen zur Gruppe $-A-(CH_2)_n-Z$ reduzierbaren Rest bedeuten und
G, Z, A und n die oben angegebene Bedeutung haben,
mit einem reduzierenden Mittel behandelt,
oder dass man eine Verbindung, die der allgemeinen Formel I entspricht, worin jedoch die Hydroxygruppe in funktionell abgewandelter Form vorliegt, mit einem solvolysierenden Mittel unter Freisetzung der Hydroxygruppe behandelt,
oder dass man eine Verbindung der allgemeinen Formel III

$R-A-(CH_2)_n-X$ III

worin
X Cl, Br, J, OH oder reaktionsfähig funktionell abgewandeltes OH bedeutet und
R, A und n die oben angegebene Bedeutung haben,
mit einer Verbindung der allgemeinen Formel H-Z umsetzt
und dass man gegebenenfalls eine erhaltene Hydroxyverbindung der Formel I, in der A $-CHOH-CHR^1-$ ist, mit einem Dehydratisierungsmittel und/oder eine erhaltene Verbindung der Formel I, in der A $-CH=CR^1-$, $-CO-CHR^1-$ oder $-CHOH-CHR^1-$ ist, mit einem reduzierenden Mittel behandelt
und/oder eine erhaltene Base der Formel I durch Behandeln mit einer Säure in eines ihrer physiologisch unbedenklichen Säureadditionssalze umwandelt.

8. Verfahren zur Herstellung pharmazeutischer Zubereitungen, dadurch gekennzeichnet, dass man eine Verbindung der Formel I und/oder eines ihrer physiologisch unbedenklichen Säureadditionssalze zusammen mit mindestens einem festen, flüssigen oder halbflüssigen Träger- oder Hilfsstoff und gegebenenfalls gin Kombination mit einem weiteren Wirkstoff in eine geeignete Dosierungsform bringt.

9. Pharmazeutische Zubereitung, gekennzeichnet durch einen Gehalt an einer Verbindung der allgemeinen Formel I und/oder einem ihrer physiologisch unbedenklichen Säureadditionssalze.

**Patentanspruch für den Vertragsstaat: AT**

Verfahren zur Herstellung von basischen Äthern der allgemeinen Formel I

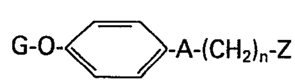 I

worin
G eine unsubstituierte oder eine ein- oder zweifach durch Halogen substituierte Phenyl- oder Benzylgruppe oder Alkyl mit 1–6 C-Atomen,
Z 1-Imidazolyl oder 2-Methyl-1-imidazolyl, $-CH=CR^1-$, $-CO-CHR^1-$, $-CHOH-CHR^1-$ oder $-CH_2-CHR^2-$,
n 1, 2 oder 3,

$R^1$ H oder Alkyl mit 1–4 C-Atomen und
$R^2$ Alkyl mit 1–4 C-Atomen
bedeuten sowie ihrer physiologisch unbedenklichen Säureadditionssalze, dadurch gekennzeichnet, dass man eine Verbindung der allgemeinen Formel II

R–Q II

worin
R die Gruppe 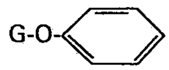 und
Q einen zur Gruppe $-A-(CH_2)_n-Z$ reduzierbaren Rest bedeuten und
G, Z, A und n die oben angegebene Bedeutung haben,
mit einem reduzierenden Mittel behandelt,
oder dass man eine Verbindung, die der allgemeinen Formel I entspricht, worin jedoch die Hydroxygruppe in funktionell abgewandelter Form vorliegt, mit einem solvolysierenden Mittel unter Freisetzung der Hydroxygruppe behandelt,
oder dass man eine Verbindung der allgemeinen Formel III

$R-A-(CH_2)_n-X$ III

worin
X Cl, Br, J, OH oder reaktionsfähig funktionell abgewandeltes OH bedeutet und
R, A und n die oben angegebene Bedeutung haben,
mit einer Verbindung der allgemeinen Formel H-Z umsetzt
und dass man gegebenenfalls eine erhaltene Hydroxyverbindung der Formel I, in der A $-CHOH-CHR^1-$ ist, mit einem Dehydratisierungsmittel und/oder eine erhaltene Verbindung der Formel I, in der A $-CH=CR^1-$, $-CO-CHR^1-$ oder $-CHOH-CHR^1-$ ist, mit einem reduzierenden Mittel behandelt und/oder eine erhaltene Base der Formel I durch Behandeln mit einer Säure in eines ihrer physiologisch unbedenklichen Säureadditionssalze umwandelt.

**Claims for the contracting states: BE, CH, DE, FR, GB, IT, NL, SE**

1. Basic ethers of the general formula I

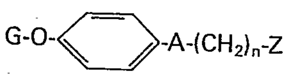 I

wherein G is an unsubstituted phenyl oder benzyl group or a phenyl or benzyl group which is monosubstituted or disubstituted by halogen or is alkyl with 1–6 C atoms, Z is 1-imidazolyl or 2-methyl-1-imidazolyl, A is $-CH=CR^1-$, $-CO-CHR^1-$, $-CHOH-CHR^1-$ or $-CH_2-CHR^2-$, n is 1, 2 or 3, $R^1$ is H or alkyl with 1–4 C atoms and $R^2$ is alkyl with 1–4 C atoms, and their physiologically acceptable acid addition salts.

2. 1-(4-p-Chlorophenoxy-phenyl)-3-(1-imidazolyl)-propene, a compound of claim 1.

3. 1-p-Phenoxyphenyl-2-methyl-3-(1-imidazolyl)-propene, a compound of claim 1.

4. 1-p-Phenoxyphenyl-2-methyl-3-(1-imidazolyl)-propane, a compound of claim 1.

5. 1-(4-p-Chlorophenoxy-phenyl)-2-methyl-3-(1-imidazolyl)-propane, a compound of claim 1.

6. 1-(4-p-Chlorophenoxy-phenyl)-2-methyl-3-(1-imidazolyl)-propene, a compound of claim 1.

7. Process for the preparation of basic ethers of the general formula I

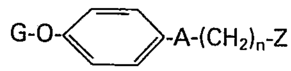 I

wherein G is an unsubstituted phenyl or benzyl group or a phenyl or benzyl group which is monosubstituted or disubstituted by halogen or is alkyl with 1–6 C atoms, Z is 1-imidazolyl or 2-methyl-1-imidazolyl, A is $-CH=CR^1-$, $-CO-CHR^1-$, $-CHOH-CHR^1-$ or $-CH_2-CHR^2-$, n is 1, 2 or 3, $R^1$ is H or alkyl with 1–4 C atoms and $R^2$ is alkyl with 1–4 C atoms, and their physiologically acceptable acid addition salts, characterised in that a compound of the general formula II

R–Q II

wherein R is the group 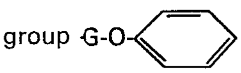 and Q is a radical which can be reduced to a group $-A-(CH_2)_n-Z$, and G, Z, A and n have the meaning indicated above, is treated with a reducing agent, or in that a compound which corresponds to the general formula I but in which the hydroxyl group is present in a funcitonally modified form is treated with a solvolysing agent with liberation of the hydroxyl group, or in that a compound of the general formula III

$R-A-(CH_2)_n-X$ III

wherein X is Cl, Br, I, OH or reactively functionally modified OH and R, A and n have the meaning indicated above, is reacted with a compound of the general formula H-Z and in that, optionally, a resulting hydroxy compound of the formula I wherein A is $-CHOH-CHR^1-$ is treated with a dehydrating agent and/or a resulting compound of the formula I wherein A is $-CH=CR^1-$, $-CO-CHR^1-$ or $-CHOH-CHR^1-$ is treated with a reducing agent and/or a resulting base of the formula I is converted to one of its physiologically acceptable acid addition salts by treatment with an acid.

8. Process for the preparation of pharmaceutical formulations, characterised in that a compound of the formula I and/or one of its physiologically acceptable acid addition salts is brought together with at least one solid, liquid or semi-liquid excipient or auxiliary and optionally in combination with a further active compound, into a suitable dosage form.

9. Pharmaceutical formulation, characterised in that it contains a compound of the general formu-

la I and/or one of its physiological acceptable acid addition salts.

**Claim for the contracting state: AT**

Process for the preparation of basic ethers of the general formula I

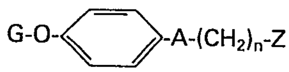 I

wherein G is an unsubstituted phenyl or benzyl group or a phenyl or benzyl group which is monosubstituted or disubstituted by halogen or is alkyl with 1–6 C atoms, Z is 1-imidazolyl or 2-methyl-1-imidazolyl, A is $-CH=CR^1-$, $-CO-CHR^1-$, $-CHOH-CHR^1-$ or $-CH_2-CHR^2-$. n is 1, 2 or 3, $R^1$ is H or alkyl with 1–4 C atoms and $R^2$ is alkyl with 1–4 C atoms, ans their physiologically acceptable acid addition salts, characterised in that a compound of the general formula II

R–Q II

wherein R is the group 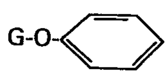 and Q is a radical which can be reduced to a group $-A-(CH_2)_n-Z$, and G, Z, A and n have the meaning indicated above, is treated with a reducing agent, or in that a compound which corresponds to the general formula I but in which the hydroxyl group is present in a functionally modified form is treated with a solvolysing agent with liberation of the hydroxyl group, or in that a compound of the general formula III

$R-A-CH_2)_n-X$ III

wherein X is Cl, Br, I, OH or reactively functionally modified OH and R, A and n have the meaning indicated above, is reacted with a compound of the general formula H-Z and in that, optionally, a resulting hydroxy compound of the formula I wherein A is $-CHOH-CHR^1-$ is treated with a dehydrating agent and/or a resulting compound of the formula I wherein A is $-CH=CR^1-$, $CO-CHR^1-$ or $-CHOH-CHR^1$ is treated with a reducing agent and/or a resulting base of the formula I is converted to one of its physiologically acceptable acid addition salts by treatment with an acid.

**Revendications pour les etats contractants: BE, CH, DE, FR, GB, IT, NL, SE**

1. Ethers basiques de formule générale I:

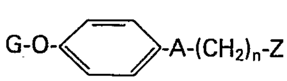 I

dans laquelle
G représente un groupe phényle ou benzyle non substitué ou substitué une ou deux fois par de l'halogène, ou un groupe alcoyle ayant 1 à 6 atomes de carbone,
Z 1-imidazolyle ou 2-méthyl-1-imidazolyle,
A $-CH=CR^1-$, $-CO-CHR^1-$, $-CHOH-CHR^1-$ ou $-CH_2-CHR^2-$,
n est 1, 2 ou 3,
$R^1$ H ou alcoyle ayant 1 à 4 atomes de carbone et
$R^2$ alcoyle ayant 1 à 4 atomes de carbone,
ainsi que leurs sels d'addition d'acides physiologiquement inoffensifs.

2. 1-(4-p-chlorophénoxyphényl)-3-(1-imidazolyl)-propène, composé selon la revendication 1.

3. 1-p-phénoxyphényl-2-méthyl-3-(1-imidazolyl)-propène, composé selon la revendication 1.

4. 1-p-phénoxyphényl-2-méthyl-3-(1-imidazolyl)-propane, composé selon la revendication 1.

5. 1-(4-p-chlorophénoxyphényl)-2-méthyl-3-(1-imidazolyl)-propane, composé selon la revendication 1.

6. 1-(4-p-chlorophénoxyphényl)-2-méthyl-3-(1-imidazolyl)-propène, composé selon la revendication 1.

7. Procédé de fabrication d'éthers basiques de formule générale I:

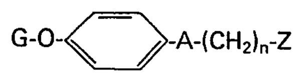 I

dans laquelle
G représente un groupe phényle ou benzyle non substitué ou substitué une ou deux fois par de l'halogène, ou alcoyle ayant 1 à 6 atomes de carbone,
Z 1-imidazolyle ou 2-méthyl-1-imidazolyle,
A $-CH=CR^1$, $-CO-CHR^1-$, $-CHOH-CHR^1$ ou $-CH_2-CHR^2-$,
n 1, 2 ou 3,
$R^1$ H ou alcoyle ayant 1 à 4 atomes de carbone et
$R^2$ alcoyle ayant 1 à 4 atomes de carbone,
de même que de leurs sels d'addition d'acides physiologiquement inoffensifs, caractérisé en ce qu'on traite un composé de formule générale:

R–Q II

dans laquelle
R signifie le groupe 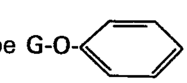 et
Q un radical réductible en groupe $-A-(CH_2)_n-Z$ et
G, Z, A et n ont la signification indiquée plus haut,
avec un agent réducteur,
ou en ce qu'on traite un composé qui correspond à la formule générale I mais dans laquelle le groupe hydroxyle se présente sous une forme fonctionnellement modifiée, avec un agent de solvolyse avec mise en liberté du groupe hydroxyle, ou en ce qu'on fait réagir un composé de formule générale III

$R-A-(CH_2)_n-X$ III

dans laquelle
X signifie Cl, Br, I, OH ou un OH fonctionnellement modifié réactif et
R, A et n ont la signification indiquée plus haut,
avec un composé de formule générale H-Z,
et en ce qu'on traite éventuellement un composé

hydroxylé obtenu de formule I dans laquelle A est $-CHOH-CHR^1$, avec un agent de déshydration et/ou un composé obtenu de formule I dans laquelle A est $-CH=CR^1-$, $-CO-CHR^1-$ ou $-CHOH-CHR^1-$, avec un agent réducteur,
et/ou en ce qu'on convertit une base obtenue de formule I par traitement avec un acide en un de ses sels d'addition d'acides physiologiquement inoffensifs.

8. Procédé de fabrication de préparations pharmaceutiques, caractérisé en ce qu'on met en une forme de dosage appropriée un composé de formule I et/ou un de ses sels d'addition d'acides physiologiquement inoffensifs conjointement avec ou moins un agent de support solide, liquide ou semi-liquide ou une substance auxiliaire et éventuellement en combinaison avec un autre matière active.

9. Préparation pharmaceutique, caractérisée par une teneur en un composé de formule générale I et/ou en un de ses sels d'addition d'acides physiologiquement inoffensifs.

**Revendication pour l'etat contractant: AT**

Procédé de fabrication d'éthers basiques de formule générale I:

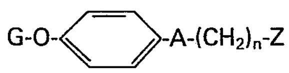 I

dans laquelle
G représente un groupe phényle ou benzyle non substitué ou substitué une ou deux fois par de l'halogène, ou alcoyle ayant 1 à 6 atomes de carbone,
Z 1-imidazolyle ou 2-méthyl-1-imidazolyle,
A $-CH=CR^1$, $-CO-CHR^1-$, $-CHOH-CHR^1$ ou $-CH_2-CHR^2-$,
n 1, 2 ou 3,
$R^1$ H ou alcoyle ayant 1 à 4 atomes de carbone et
$R^2$ alcoyle ayant 1 à 4 atomes de carbone,
de même que de leurs sels d'addition d'acides physiologiquement inoffensifs, c aractérisé en ce qu'on traite un composé de formule générale:

R–Q II

dans laquelle
R signifie le groupe 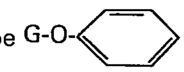 et
Q un radical réductible en groupe $-A-(CH_2(_n-Z$ et
G, Z, A et n ont la signification indiquée plus haut,
avec un agent réducteur,
ou en ce qu'on traite un composé qui correspond à la formule générale I mais dans laquelle le groupe hydroxyle se présente sous une forme fonctionnellement modifiée, avec un agent de solvolyse avec mise en liberté du groupe hydroxyle, ou en ce qu'on fait réagir un composé de formule générale III

$R-A-(CH_2)_n-X$ III

dans laquelle
X signifie Cl, Br, I, OH ou un OH fonctionnellement modifié réactif et
R, A et n ont la signification indiquée plus haut,
avec un composé de formule générale H-Z,
et en ce qu'on traite éventuellement un composé hydroxylé obtenu de formule I dans laquelle A est $-CHOH-CHR^1$, avec un agent de déshydratation et/ou un composé obtenu de formule I dans laquelle A est $-CH=CR^1-$, $-CO-CHR^1-$ ou $-CHOH-CHR^1-$, avec un agent réducteur,
et/ou en ce qu'on convertit une base obtenue de formule I par traitement avec un acide en un de ses sels d'addition d'acides physiologiquement inoffensifs.